(19) Europäisches Patentamt
European Patent Office
Office européen des brevets

(11) Veröffentlichungsnummer: **0 119 539**
**A2**

(12) # EUROPÄISCHE PATENTANMELDUNG

(21) Anmeldenummer: **84102360.9**

(22) Anmeldetag: **05.03.84**

(51) Int. Cl.³: **C 07 H 15/12**, A 01 N 43/16

(30) Priorität: **12.03.83 DE 3308806**

(43) Veröffentlichungstag der Anmeldung: **26.09.84 Patentblatt 84/39**

(84) Benannte Vertragsstaaten: **AT BE CH DE FR GB IT LI NL SE**

(71) Anmelder: **BASF Aktiengesellschaft, Carl-Bosch-Strasse 38, D-6700 Ludwigshafen (DE)**

(72) Erfinder: **Kropp, Rudolf, Sprottauer Strasse 2, D-6703 Limburgerhof (DE)**
Erfinder: **Fischer, Martin, Dr., Elbingerweg 1, D-6700 Ludwigshafen 29 (DE)**
Erfinder: **Ammermann, Eberhard, Dr., Sachsenstrasse 3, D-6700 Ludwigshafen (DE)**
Erfinder: **Pommer, Ernst-Heinrich, Dr., Berliner Platz 7, D-6703 Limburgerhof (DE)**

(54) **Fungizide Mittel, substituierte Glucopyranosylamine und Verfahren zur Bekämpfung von Pilzen.**

(57) Fungizide Mittel, enthaltend einen inerten Trägerstoff und eine Verbindung der allgemeinen Formel

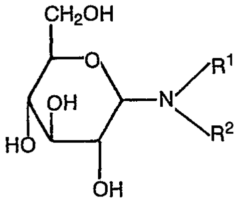

worin $R^1$ und $R^2$ Wasserstoff, einen substituierten Alkyl- oder Alkenylrest, einen gegebenenfalls substituierten Cycloalkylrest, einen gegebenenfalls substituierten Aralkylrest sowie einen gegebenenfalls substituierten Arylrest bedeuten und darüber hinaus $R^1$ und $R^2$ Glieder eines gemeinsamen 5- oder 6-gliedrigen Ringes sein können, der noch weitere Heteroatome enthalten kann, D-Glucopyranosyl-1-amine und Verfahren zur Bekämpfung von Pilzen.

EP 0 119 539 A2

ACTORUM AG

Fungizide Mittel, substituierte Glucopyranosylamine und Verfahren zur Bekämpfung von Pilzen

Die vorliegende Erfindung betrifft substituierte Glucopyranosylamine, diese enthaltende fungizide Mittel und Verfahren zur Bekämpfung von Pilzen, insbesondere pflanzenpathogenen Pilzen.

Die fungizide Wirkung von N-Trichlormethylthiophthalimid ist bekannt (R. Wegler, "Chemie der Pflanzenschutz- und Schädlingsbekämpfungsmittel", Bd. 4, Seite 191, Springer-Verlag, Berlin/Heidelberg/New York, 1977).

Es wurde gefunden, daß Glucopyranosylamine der allgemeinen Formel

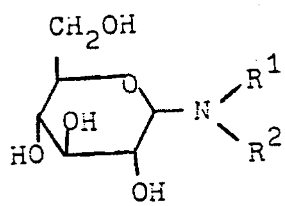

worin $R^1$ und $R^2$ gleich oder verschieden sind und Wasserstoff, einen gegebenenfalls durch eine substituierte Aminogruppe oder Phenyl oder Hydroxyl substituierten Alkyl- oder Alkenylrest mit 1 oder 2 bis 20 Kohlenstoffatomen, einen gegebenenfalls durch Halogen, Nitro oder $C_1$-$C_2$-Alkoxy substituierten Cycloalkylrest mit 3 bis 6 Kohlenstoffatomen im Ring, einen gegebenenfalls durch Halogen im Arylteil substituierten Aralkylrest mit 7 bis 15 Kohlenstoffatomen sowie einen gegebenenfalls durch Hydroxy, Halogen, Nitro oder $C_1$-$C_2$-Alkoxy substituierten Arylrest bedeuten und darüber hinaus $R^1$ und $R^2$ Glieder eines gemeinsamen 5- oder 6-gliedrigen Ringes sein können, der noch weitere Heteroatome enthalten kann, eine gute fungizide Wirkung besitzen.

Alkyl mit 1 bis 20 C-Atomen ist beispielsweise Octadecyl, Hexadecyl, Tridecyl, Dodecyl, Octyl, Hexyl, Pentyl und insbesondere Alkyl mit 1 bis 4 C-Atomen, z.B. Methyl, Ethyl, Propyl, Butyl. Alkenyl mit 2 bis 20 C-Atomen ist beispielsweise Propenyl oder Oleyl. Cycloalkyl mit 3 bis 6 Kohlenstoffatomen ist beispielsweise Cyclopropyl, Cyclopentyl, Cyclohexyl.

Aralkyl mit 7 bis 15 C-Atomen ist beispielsweise Benzyl, Phenylethyl, Phenylpropyl.

Halogen ist insbesondere Chlor oder Brom.

Sws/ro

Aryl ist beispielsweise Naphthyl und insbesondere Phenyl. Ein Heteroatome enthaltender 5- oder 6-gliedriger Ring ist beispielsweise ein Stickstoff, Sauerstoff oder Schwefel enthaltender Ring, beispielsweise Piperidin, Morpholin, Triazol, Imidazol, Pyrrolidin, Thiomorpholin, N'-Methyl--piperazin. Alkyloxy mit 1 bis 2 Kohlenstoffatomen ist Methoxy, Ethoxy.

Als bevorzugte Wirkstoffe seien genannt:

| Nr. | | | | Fp. (in °C) |
|---|---|---|---|---|
| 1 | D-Gluco-pyranosyl-1-amin | | | 126 - 127 |
| 2 | " | " | -N-pentyl-amin | 96 - 97 |
| 3 | " | " | -N-octyl-amin | 100 - 104 |
| 4 | " | " | -N-dodecyl-amin | 104 - 106 |
| 5 | " | " | -N-tridecyl-amin | 109 - 113 |
| 6 | " | " | -N-octadecyl-amin | 103 - 105 |
| 7 | " | " | -N-oleyl-amin | 92 - 95 |
| 8 | " | " | -N,N-di(ß-hydroxy--ethyl)amin | 123 - 125 |
| 9 | " | " | -piperidin | 125 - 126 |
| 10 | " | " | -2,6-dimethyl-morpholin | 129 - 131 |
| 11 | " | " | -imidazol | 203 (Z) |
| 12 | " | " | -2,5-dichloranilin | 149 - 150 |
| 13 | " | " | -N-(3,4-dichlor-benzyl)--amin | 130 - 133 |
| 14 | " | " | -N-(2,4-dichlor-benzyl)--amin | 134 - 137 |
| 15 | " | " | -N-[2-methyl-3-(p-tert. butyl-phenyl)-propyl]--amin | $[\alpha]_D^{20} = -1,8°$ (c = 2, Ethanol) |
| 16 | " | " | -N-(3-phenyl-propyl)--amin | $[\alpha]_D^{20} = -4,5°$ (c = 2, Ethanol) |
| 17 | " | " | -N,N-dibenzylamin | 139 - 141 |
| 18 | " | " | -N-3[2'-methyl-3'-(p--tert.butyl-phenyl)--propyl)-amin]-propyl amin der Formel | $[\alpha]_D^{20} = -1,1°$ (c = 2, Ethanol) Öl |

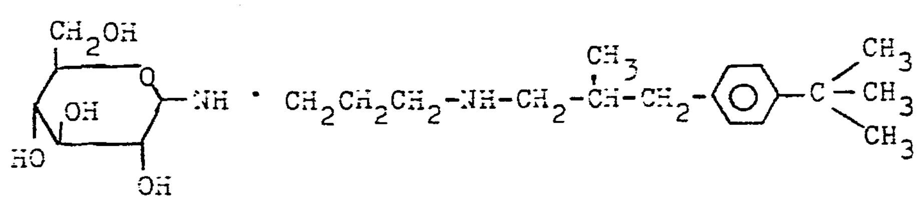

Die Herstellung der Wirkstoffe erfolgt nach bekannten Methoden aus D-Glucose oder ihren Derivaten (G. P. Ellis und J. Honeyman, Advances in Carbohydrate Chemistry 10/95 bis 168 (1955)).

Beispiel 1

D-Gluco-pyranosyl-N-dodecyl-amin

198 g D-Glucose 1 $H_2O$ wurden mit 185 g Dodecylamin in 2 l Ethanol 24 Stunden bei Raumtemperatur (20°C) gerührt. Anschließend wurde noch 1 Stunde bei 50°C gerührt. Nach dem Abkühlen und Absaugen erhielt man 330 g (= 95 % d.Th.) D-Gluco-pyranosyl-1-N-dodecyl-amin (Verbindung Nr. 4) vom Fp. 102 bis 106°C. Nach dem Umkristallisieren aus Ethanol liegt der Schmelzpunkt bei 104 bis 106°C. Elementaranalyse: ($C_{18}H_{37}O_5N$)

| | C | H | O | N |
|---|---|---|---|---|
| gef. | 62,4 % | 10,5 % | 23,3 % | 4,0 % |
| ber. | 62,2 % | 10,7 % | 23,0 % | 4,7 % |

Beispiel 2

D-Gluco-pyranosyl-N,N-di-(ß-hydroxy-ethyl)-amin

36 g D-Glucose, wasserfrei, wurden mit 34 g Diethanolamin 10 Min. bei 80 bis 90°C gerührt. Es wurden 50 ml Ethanol zugegeben und weitere 40 Min. bei 70 bis 80°C gerührt. Anschließend gab man nochmals 50 ml Ethanol zu, versetzte mit wenig Aktivkohle und filtrerte. Das Filtrat wurde mit 50 ml Di-ethylether versetzt. Dabei scheidet sich ein Öl ab, das beim Stehen kristallisiert. Man erhielt 32 g (60 % der Theorie) D-Gluco-pyranosyl-N,N-di-(ß-hydroxyethyl)-ami n (Verbindung Nr. 8) vom Fp. 113 bis 115°C. Nach dem Umkristallisieren aus Methanol schmilzt die Substanz bei 123 bis 125°C.

Elementaranalyse ($C_{10}H_{21}O_7N$)

| | C | H | O | N |
|---|---|---|---|---|
| gef. | 44,6 % | 7,8 % | 42,3 % | 5,3 % |
| ber. | 44,9 % | 7,9 % | 41,9 % | 5,2 % |

Beispiel 3

N-(D-Gluco-pyranosyl)-imidazol

97 g  -Brom-tetraacetyl-D-glucose werden mit 16,5 g Imidazol und 23,6 g Triethylamin in 500 ml Acetonitril 3 Stunden zum Sieden erhitzt. Nach dem Abkühlen wird vom entstandenen Triethylammoniumbromid abgesaugt und das

Filtrat eingeengt. Es kristallisieren dabei 11 g N-(Tetraacetyl-D-gluco-pyranosyl)-imidazol mit einem Schmelzpunkt von 191 bis 196°C aus.

Die Substanz wurde in 200 ml einer 5 Gew.%igen methanolischen Ammoniak-Lösung 48 Stunden bei Zimmertemperatur (20°C) gerührt. Die Lösung wurde eingeengt und der Rückstand mit Aceton gewaschen. Man erhielt 6 g (11 % der Theorie) N-(D-Gluco-pyranosyl)-imidazol (Verbindung Nr. 11), das bei 203°C unter Zersetzung schmilzt.

Die Wirkstoffe zeigen eine starke fungitoxische Wirksamkeit, insbesondere gegen phytopathogene Pilze. Sie dienen zur Verhütung und Heilung von Pflanzenkrankheiten, die durch Mikroorganismen verursacht werden, wie z.B. Botrytis cinerea, Plasmopara viticola, Monilia fructigena, Alternaria solani, Sclerotinia sclerotiorum, Pyricularia oryzae, Pellicularia sasakii, Erysiphe graminis, Erysiphe cichoracearum, Chaetomium globosum, Aspergillus niger, Xanthomonas oryzae, Xanthomonas citri, Phytophthora infestans (an Kartoffeln und Tomaten).

Die Wirkstoffe können gleichzeitig das Wachstum von zwei oder mehr der genannten Pilze unterdrücken und besitzen eine hohe Pflanzenverträglichkeit. Ein Teil der Wirkstoffe zeigt kurative Eigenschaften, d.h. die Anwendung der Mittel kann noch nach erfolgter Infektion der Pflanzen durch die Krankheitserreger vorgenommen werden, um einen sicheren Bekämpfungserfolg zu erzielen.

Die fungiziden Mittel enthalten 0,1 bis 95 % (Gewichtsprozent) Wirkstoff, vorzugsweise 0,5 bis 90 %. Die Aufwandmengen liegen je nach Art des gewünschten Effektes zwischen 0,1 bis 5 kg Wirkstoff je ha.

Die Wirkstoffe können auch zusammen mit anderen Wirkstoffen, z.B. Herbiziden, Insektiziden, Wachstumsregulatoren und anderen Fungiziden, oder auch mit Düngemitteln vermischt ausgebracht werden. In vielen Fällen erhält man bei der Mischung mit Fungiziden auch eine Vergrößerung des fungiziden Wirkungsspektrums; bei einer Anzahl dieser Fungizidmischungen treten auch synergistische Effekte auf, d.h. die fungizide Wirksamkeit des Kombinationsproduktes ist größer als die der addierten Wirksamkeiten der Einzelkomponenten. Eine besonders günstige Vergrößerung des Wirkungsspektrums wird mit folgenden Fungiziden erzielt:

Schwefel,
Dithiocarbamate und deren Derivate, wie
Ferridimethyldithiocarbamat
Zinkdimethyldithiocarbamat

Zinkethylenbisdithiocarbamat
Manganethylenbisdithiocarbamat
Mangan-Zink-ethylendiamin-bis-dithiocarbamat
Tetramethylthiuramdisulfide
Ammoniak-Komplex von Zink-(N,N'-ethylen-bis-dithiocarbamat)
Ammoniak-Komplex von Zink-(N,N'-propylen-bis-dithiocarbamat) Zink-(N,N'--propylen-bis-dithiocarbamat)
N,N'-Polypropylen-bis-(thiocarbamoyl)-disulfid

Nitroderivate, wie
Dinitro-(1-methylheptyl)-phenylcrotonat
2-sec.-Butyl-4,6-dinitrophenyl-3,3-dimethylacrylat
2-sec.-Butyl-4,6-dinitrophenyl-isopropylcarbonat
5-Nitro-isophthalsäure-di-isopropylester

heterocyclische Strukturen, wie
2-Heptadecyl-2-imidazolin-acetat
2,4-Dichlor-6-(o-chloranilino)-s-triazin
O,O-Diethyl-phthalimidophosphonothioat
5-Amino-1-(bis-(dimethylamino)-phosphinyl)-3-phenyl-1,2,4-triazol)
2,3-Dicyano-1,4-dithiaanthrachinon
2-Thio-1,3-dithio-(4,5,6)-chinoxalin
1-(Butylcarbamoyl)-2-benzimidazol-carbaminsäuremethylester
2-Methoxycarbonylamino-benzimidazol
2-(Furyl-(2)-benzimidazol
2-(Thiazolyl-(4)-benzimidazol
N-(1,1,2,2-Tetrachlorethylthio)-tetrahydrophthalimid
N-Trichlormethylthio-tetrahydrophthalimid
N-Trichlormethyl-phthalimid
N-Dichlorfluormethylthio-N',N'-dimethyl-N-phenyl-schwefelsäurediamid
5-Ethoxy-3-trichlormethyl-1,2,3-thiadiazol
2-Rhodanmethylthiobenzthiazol
1,4-Dichlor-2,5-dimethoxybenzol
4-(2-Chlorphenylhydrazono)-3-methyl-5-isoxazolon
Pyridin-2-thio-1-oxid
8-Hydroxychinolin bzw. dessen Kupfersalz
2,3-Dihydro-5-carboxanilido-6-methyl-1,4-oxathiin-4,4-dioxid
2,3-Dihydro-5-carboxanilido-6-methyl-1,4-oxathiin
2-Methyl-5,6-dihydro-4H-pyran-3-carbonsäure-anilid
2-Methyl-furan-3-carbonsäureanilid
2,5-Dimethyl-furan-3-carbonsäureanilid
2,4,5-Trimethyl-furan-3-carbonsäureanilid
2,5-Dimethyl-furan-3-carbonsäurecyclohexylamid

N-Cyclohexyl-N-methoxy-2,5-dimethyl-furan-3-carbonsäureamid
2-Methyl-benzoesäure-anilid
2-Jod-benzoesäure-anilid
N-Formyl-N-morpholin-2,2,2-trichlorethylacetal
Piperazin-1,4-diylbis-(1-(2,2,2-trichlor-ethyl)-formamid
1-(3,4-Dichloranilino)-1-formylamino-2,2,2-trichlorethan
2,6-Dimethyl-N-tridecyl-morpholin bzw. dessen Salze
2,6-Dimethyl-N-cyclododecyl-morpholin bzw. dessen Salze
N-[3-(p-tert.-Butylphenyl)-2-methylpropyl]-cis-2,6-dimethylmorpholin
N-[3-(p-tert.-Butylphenyl)-2-methylpropyl]-piperidin
1-[2-(2,4-Dichlorphenyl)-4-ethyl-1,3-dioxolan-2-yl-ethyl]-1H-1,2,4-triazol
1-[2-(2,4-Dichlorphenyl)-4-n-propyl-1,3-dioxolan-2-ylethyl]-1H-1,2,4-triazol
N-(n-Propyl)-N-(2,4,6-trichlorphenoxyethyl)-N'-imidazol-yl-harnstoff
1-(4-Chlorphenoxy)-3,3-dimethyl-1-(1H-1,2,4-triazol-1-yl) -(2-Chlorphenyl)- -(4-chlorphenyl)-5-pyrimidin-methanol
5-Butyl-2-dimethylamino-4-hydroxy-6-methyl-pyrimidin
Bis-(p-chlorphenyl)-3-pyridinmethanol
1,2-Bis-(3-ethoxycarbonyl-2-thioureido)-benzol
1,2-Bis-(3-methoxycarbonyl-2-thioureido)-benzol

ssowie verschiedene Fungizide, wie
Dodecylguanidinacetat
3-(3-(3,5-Dimethyl-2-oxycyclohexyl)-2-hydroxyethyl)-glutarimid
Hexachlorbenzol
DL-Methyl-N-(2,6-dimethyl-phenyl)-N-furoyl(2)-alaninat,
DL-N-(2,6-Dimethyl-phenyl)-N-(2'-methoxyacetyl)-alanin-methylester,
N-(2,6-Dimethylphenyl)-N-chloracetyl-D,L-2-aminobutyrolacton,
DL-N-(2,6-Dimethylphenyl)-N-(phenylacetyl)-alaninmethylester
5-Methyl-5-vinyl-3-(3,5-dichlorphenyl)-2,4-dioxo-1,3-oxazolidin
3-(3,5-Dichlorphenyl)-5-methyl-5-methoxymethyl-1,3-oxazolidin-2,4-dion
3-(3,5-Dichlorphenyl)-1-isopropylcarbamoylhydantoin
N-(3,5-Dichlorphenyl)-1,2-dimethylcyclopropan-1,2-dicarbonsäureimid
2-Cyano-N-(ethylaminocarbonyl)-2-methoximino-acetamid
2-(Thiocyanomethylthio)-benzthiazol
1-[2-(2,4-Dichlorphenyl)-4-ethyl-1,3-dioxolan-2-yl-methyl]-1H-1,2,4-triazol
2,4,5,6-Tetrachlor-isophthalodinitril
Methylenbisthiocyanat
Tributylzinnoxid
Mercaptobenzthiazol
Benzisothiazolon und seine Alkalisalze

Alkaliverbindungen des N'-Hydroxy-N-cyclohexyl-diazeniumoxids
2-(Methoxy-carbonylamino)-benzimidazol
2-Methyl-3-oxo-5-chlor-thiazolin-3-on
Trihydroxymethyl-nitro-methan
Glutardialdehyd
Chloracetamid

Die Wirkstoffe werden beispielsweise in Form von direkt versprühbaren Lösungen, Suspensionen, Emulsionen, auch in Form von hochprozentigen wäßrigen, öligen oder sonstigen Dispersionen, Pasten, Stäubemitteln, Streumitteln, Granulaten durch Versprühen, Verstäuben, Verstreuen, Verstreichen oder Gießen ausgebracht. Die Anwendungsformen richten sich ganz nach den Verwendungszwecken; sie sollten in der Regel möglichst eine feine Verteilung der Wirkstoffe gewährleisten.

Zur Herstellung von direkt oder nach Emulgieren in Wasser verwendbaren Lösungen, Emulsionen, Pasten und Öldispersionen kommen Mineralölfraktionen von mittlerem bis hohem Siedepunkt, wie Kerosin oder Dieselöl, ferner Kohlenteeröle usw., sowie Öle pflanzlichen oder tierischen Ursprungs, aliphatische, cyclische oder aromatische Kohlenwasserstoffe, zum Beispiel Benzol, Toluol, Xylol, Paraffin, Tetrahydronaphthalin, alkylierte Naphthaline oder deren Derivate z.B. Methanol, Ethanol, Propanol, Butanol, Chloroform, Tetrachlorkohlenstoff, Cyclohexanol, Cyclohexanon, Chlorbenzol, Isophoron usw., stark polare Lösungsmittel, wie z.B. Dimethylformamid, Dimethylsulfoxid, N-Methylpyrrolidon, Wasser usw. in Betracht. Bevorzugt wird die Lösung in Wasser.

Wäßrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäuren, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylen-octylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Phospolipide Sulfitablaugen und Methylcellulose in Betracht.

Pulver, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden wie Silicagel, Kieselsäuren, Kieselgele, Silikate, Talkum, Kaolin, Attaclay, Kalkstein, Kreide, Talkum, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehle, Baumrinden-, Holz- und Nußschalenmehl, Cellulosepulver und andere feste Trägerstoffe.

Beispiele für solche Pflanzenschutzmittel-Zubereitungen sind:

I. Man vermischt 90 Gewichtsteile der Verbindung 4 mit 100 Gewichtsteilen N-Methylpyrrolidon und erhält eine Lösung, die zur Anwendung in Form kleinster Tropfen geeignet ist.

II. 20 Gewichtsteile der Verbindung 6 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Doedecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in Wasser erhält man eine wäßrige Dispersion.

III. 20 Gewichtsteile der Verbindung 13 werden in einer Mischung gelöst, die aus 30 Gewichtsteilen Cyclohexanon, 30 Gewichtsteilen Isobutanol, 20 Gewichtsteilen des Anlagerungsproduktes von 40 Mol Ethylenoxid an 1 Mol Ricinusöl besteht. Durch Eingießen und Verrühren der Lösung in Wasser erhält man eine wäßrige Dispersion.

IV. 20 Gewichtsteile der Verbindung 14 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und Verrühren der Lösung in Wasser, erhält man eine wäßrige Dispersion.

V. 20 Gewichtsteile der Verbindung 16 werden mit 3 Gewichtsteilen des Natriumsalzes der Diisobutylnaphthalin-alpha-sulfonsäure, 17 Gewichtsteilen des Natriumsalzes einer Ligninsulfonsäure aus einer Sulfitablauge und 60 Gewichtsteilen pulverförmigem Kieselsäuregel gut vermischt und in einer Hammermühle vermahlen. Durch feines Verteilen der Mischung in Wasser erhält man eine Spritzbrühe.

VI. 5 Gewichtsteile der Verbindung 4 werden mit 95 Gewichtsteilen feinteiligem Kaolin innig vermischt. Man erhält auf diese Weise ein Stäubemittel, das 5 Gew.-% des Wirkstoffs enthält.

VII. 30 Gewichtsteile der Verbindung 6 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gewichtsteile der Verbindung 13 werden mit 30 Teilen Natriumsalz eines Phenolsulfonsäure-Harnstoff-Formaldehyd-Kondensates, 2 Teilen Kieselgel und 48 Teilen Wasser innig vermischt. Man erhält eine stabile wäßrige Dispersion.

IX. 20 Teile der Verbindung 14 werden mit 2 Teilen Calciumsalz der Dodecylbenzolsulfonsäure, 8 Teilen Fettalkoholpolyglykolether, 2 Teilen Natriumsalz eines Phenolsulfonsäure-harnstoff-formaldehyd-Kondensates und 68 Teilen eines paraffinischen Mineralöls innig vermischt. Man erhält eine stabile ölige Dispersion.

Die folgenden Versuche erläutern die biologische Wirkung der Verbindungen. Vergleichsmittel (A) ist der bekannte Wirkstoff N-Trichlormethylthio-phthalimid.

Versuch 1

Wirksamkeit gegen Plasmopara viticola

Blätter von Topfreben der Sorte "Müller-Thurgau" werden mit wäßriger Spritzbrühe, die 80 % (Gew.%) Wirkstoff und 20 % Emulgiermittel in der Trockensubstanz enthält, besprüht. Um die Wirkungsdauer der Wirkstoffe beurteilen zu können, werden die Pflanzen nach dem Antrocknen des Spritzbelages 10 Tage im Gewächshaus aufgestellt. Erst dann werden die Blätter mit einer Zoosporenaufschwemmung von Plasmopara viticola (Rebenperonospora) infiziert. Danach werden die Reben zunächst für 16 Stunden in einer wasserdampfgesättigten Kammer bei 24°C und anschließend für 8 Tage in einem Gewächshaus mit Temperaturen zwischen 20 und 30°C aufgestellt. Nach dieser Zeit werden die Pflanzen zur Beschleunigung des Sporangienträgerausbruches abermals für 16 Stunden in der feuchten Kammer aufgestellt. Dann erfolgt die Beurteilung des Ausamßes des Pilzausbruches auf den Blattunterseiten.

Das Ergebnis des Versuches zeigt, daß beispielsweise die Verbindung Nr. 4 bei der Anwendung als 0,025 %ige (Gew.%) Spritzbrühe eine bessere fungizide Wirkung (beispielsweise 97 %) zeigt als der Wirkstoff A (90 %).

Versuch 2

Wirksamkeit gegen Weizenmehltau

Blätter von in Töpfen gewachsenen Weizenkeimlingen der Sorte "Jubilar" werden mit wäßriger Spritzbrühe, die 80 % Wirkstoff und 20 % Emulgiermittel in der Trockensubstanz enthält, besprüht und 24 Stunden nach dem Antrocknen des Spritzbelages mit Oidien (Sporen) des Weizenmehltaus (erysiphe graminis var. tritici) bestäubt. Die Versuchspflanzen werden anschließend im Gewächshaus bei Temperaturen zwischen 20 und 22°C und 75 bis 80 % relativer Luftfeuchtigkeit aufgestellt. Nach 7 Tagen wird das Ausmaß der Mehltauentwicklung ermittelt.

Das Ergebnis des Versuches zeigt, daß beispielsweise die Verbindungen Nr. 6, 13, 14 und 16 bei der Anwendung als 0,025 %ige Spritzbrühe eine sehr gute fungizide Wirkung (beispielsweise 97 %) zeigen.

Patentansprüche

1. Fungizide Mittel, enthaltend einen inerten Trägerstoff und eine Verbindung der allgemeinen Formel

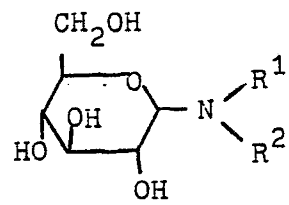

worin $R^1$ und $R^2$ gleich oder verschieden sind und Wasserstoff, einen gegebenenfalls durch eine substituierte Aminogruppe oder Phenyl oder Hydroxyl substituierten Alkyl- oder Alkenylrest mit 1 oder 2 bis 20 Kohlenstoffatomen, einen gegebenenfalls durch Halogen, Nitro oder $C_1$-$C_2$-Alkoxy substituierten Cycloalkylrest mit 3 bis 6 Kohlenstoffatomen im Ring, einen gegebenenfalls durch Halogen im Arylteil substituierten Aralkylrest mit 7 bis 15 Kohlenstoffatomen sowie einen gegebenenfalls durch Hydroxy, Halogen, Nitro oder $C_1$-$C_2$-Alkoxy substituierten Arylrest bedeuten und darüber hinaus $R^1$ und $R^2$ Glieder eines gemeinsamen 5- oder 6-gliedrigen Ringes sein können, der noch weitere Heteroatome enthalten kann.

2. Fungizid gemäß Anspruch 1, dadurch gekennzeichnet, daß $R^1$ einen Alkylrest mit 3 bis 20 Kohlenstoffatomen, der gegebenenfalls durch Phenyl substituiert ist, oder einen Halogenbenzylrest und $R^2$ Wasserstoff bedeutet.

3. Verbindungen der Formel

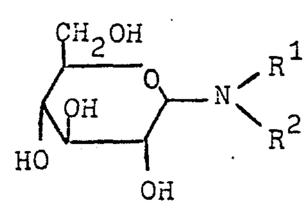

in der $R^1$ einen Tridecyl- oder Oleylrest, einen Phenylpropyl- oder Halogenbenzylrest, einen durch tert. Butylphenyl substituierten Butylrest oder einen Propylaminrest bedeutet, dessen Aminrest substituiert ist durch einen Butylrest, der durch tert. Butylphenyl substituiert

ist, und $R^2$ Wasserstoff bedeutet oder $R^1$ und $R^2$ zusammen mit dem Stickstoffatom, dessen Substituenten sie sind, einen Dimethylmorpholinrest bedeuten.

4. Verfahren zur Bekämpfung von Pilzen, dadurch gekennzeichnet, daß man die Pilze oder die von Pilzbefall bedrohten Pflanzen, Flächen oder Saatgüter mit einer Verbindung gemäß Anspruch 1 behandelt.

5. D-Gluco-pyranosyl-N-tridecylamin.

6. N-(D-Gluco-pyranosyl)-2,6-dimethylmorpholin.

7. D-Gluco-pyranosyl-N-(3,4-dichlorbenzyl)-amin.

8. D-Gluco-pyranosyl-N-(2,4-dichlorbenzyl)-amin.

9. D-Gluco-pyranosyl-N-(3-phenyl-propyl)-amin.